# EUROPEAN PATENT APPLICATION

(11) **EP 4 382 112 A1**
(43) Date of publication of application: **12.06.2024**
(21) Application number: 22866419.9
(22) Date of filing: 24.08.2022
(51) Int. Cl.: A61K 35/74, C12N 15/00, C12N 5/071

(54) **METHOD FOR BUILDING EYE DISEASE MODEL AND APPLICATION THEREOF**

(30) Priority: 09.09.2021 CN 202111057929
(71) Applicant: Smilebiotek Zhuhai Limited, Zhuhai City, Guangdong Province 519000 (CN)
(72) Inventor: WEI, Lai, Zhuhai, Guangdong 519000 (CN)
(74) Representative: Murgitroyd & Company
(86) International application number: PCT/CN2022/114537
(87) International publication number: WO 2023/035949

(57) **Abstract**

Provided is a method for building an eye disease model, comprising infecting microorganisms onto a model carrier. Also provided is an eye model carrier infected with microorganisms prepared using said method. The eye model carrier can be used for eye disease research and eye disease drug screening. The eye disease refers to retinal degeneration and the microorganisms refer to intestinal bacteria or bacteria identical to the intestinal bacteria.

## Description

### FIELD OF THE INVENTION

The present invention belongs to the technical field of diagnosis and treatment of ophthalmic diseases, and in particular relates to a method for building an eye disease model, and application of the eye disease model obtained by the method and a model carrier in eye disease research and drug screening.

### BACKGROUND ART

Inherited retinal degeneration (IRD) is a group of inherited diseases that are the most common cause of reduced vision in labor populations in industrialized countries. Inherited retinal degeneration (IRD) is a group of inherited diseases characterized by progressive loss of photoreceptor cells, including Leber congenital amaurosis (LCA), retinitis pigmentosa (RP), early-onset rod-cone dystrophy, rod-rod dystrophy, congenital stationary night blindness and color blindness, and Stargardt's disease (Broadgate et al., 2017). It is the most common cause of vision loss in working populations in industrialized countries, with an estimated incidence of 1:2000 (Kutluer et al., 2020). Due to genetic and clinical heterogeneity, treatment of IRDs requires highly personalized therapeutic strategies. Although neuroprotection, gene therapy and cell replacement therapy are proposed methods for treatment of IRD at different stages, so far, only one gene therapy (Luxtuma) that corrects mutations in an RPE65 gene is approved by FDA for treatment of LCA (Botto et al., 2021; Ikelle et al., 2020; Kutluer et al., 2020).

More than 200 genes are related to IRD. A CRB1 gene has long been recognized as an important gene, and its mutations result in a variety of ophthalmic phenotypes, including LCA and RP (Ehrenberg et al., 2013). CRB1, as a key regulatory factor of adhesion molecule connectivity, plays an important role in establishing cell polarity and maintaining eye barrier integrity. The frequency of CRB1 mutations caused by IRD varies greatly worldwide. For example, the allele frequency of CRB1 mutations found in LCA cases is 6.7% in the United States, 1.7% in Canada, 0% in the Netherlands, 16.7% in Spain, and 11.5% in China (Li et al., 2011; Vallespin et al., 2007; Zemant et al., 2005), while CRB1 is a gene with the highest mutation frequency found in all LCA, early-onset RP, and non-early-onset RP patients in Spain (Vallespin et al., 2007). On the whole, CRB1 gene mutations account for 4% of RP and 10-15% of LCA worldwide (Richard et al., 2006). Thus, there is an urgent need for a treatment method to rescue CRB1-related vision loss.

### BRIEF SUMMARY OF THE INVENTION

An object of the present invention is to provide a method for building an eye disease model or an eye disease model carrier, in particular a method for building a retinal degeneration model or a model carrier, and a model or a model carrier built by the method, as well as application of the method, the disease model and/or the disease model carrier in eye disease reaseach and related drug screening.

The object of the present invention is achieved by the following technical solutions:
the present invention provides a method for building an eye disease model, including infecting the eye disease model with a microorganism.

Preferably, the infecting includes direct contact with the microorganism or indirect contact with the microorganism. In one specific embodiment, an eye disease model in which an eye is infected with a microorganism is obtained by raising a non-human animal in a specific pathogen-free (SPF) environment.

Preferably, the microorganism is derived from intestinal bacteria of the same individual or is the same as the intestinal bacteria of the individual.

Preferably, the eye disease includes retinal degeneration; more preferably, the retinal degeneration is progressive retinal degeneration.

Preferably, the retinal degeneration is inherited retinal degeneration (IRD).

Preferably, the eye disease includes LCA, RP, arRP, EORD, EORP, PPRPE, rettelangiectasia and/or choroideremia like fundus.

Preferably, the eye disease includes ocular inflammation, e.g., uveitis, glaucoma, and age-related macular degeneration (AMD), vitritis, choroiditis, retinitis, retinal vasculitis, optic neuritis, as well as uveitis, Behcet's disease, Vogt-Koyanagi-Harada syndrome, uveitis, retinopathy, sympathetic ophthalmia, cataract, conjunctivitis, glaucoma, and the like.

Preferably, the model is a non-human animal, preferably a monkey, a dog, a chimpanzee, a rat, and a mouse.

Preferably, the model carrier is a cell, a tissue or an organ, and the cell, the tissue or the organ is derived from a human or a non-human animal.

Preferably, the cell is a primary cell or a cell line.

Preferably, the tissue is an ocular tissue and the organ is an ocular organ.

Preferably, the tissue or the organ is a regenerative tissue or organ.

Preferably, the model has a disease-causing mutation in a gene.

Preferably, the gene with the disease-causing mutation is a gene related to maintaining a structure of a retinal barrier, the retinal barrier being an outer blood-retinal barrier and/or an inner blood-retinal barrier.

Preferably, a disease-causing mutation of an eye disease-related gene is present in the model or the model carrier, and the gene with the disease-causing mutation of an ocular gene is selected from one or a combination of two or more of of the following genes: ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

In one specific embodiment, the eye disease-related gene with the disease-causing mutation in the model or the model carrier includes the CRB1 gene.

Preferably, a mutation of the CRB1 gene of the model or the model carrier includes one or two or more of the following mutations: c.107C>G, c.11 IdelT, c.135C>G, c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G>A, c.3103C>T, c.4082G> A, c.4060G>A, c.866C>T, c.1463T>C, c.2035C>G, c.2306_2307GC>AG, c.2306G>A, c.2714G>A, c.2875G>A and c.3992G>A.

Further preferably, the mutation of the CRB1 gene of the model or the model carrier includes one or two or more of the following mutations: c.4006-1G>T, c.3686G>C, (p.Cys1229Ser), c.2842+1delinsAA, c.4060G>A, (p.Ala1354Thr), c.3991C>T, (p.Arg1331Cys), c.3014A>T, (p.Asp1005Val), c.4005+1G>A, c.2680_2684del, (p.Asn894fs), c.1733T>A, (p.Val578Glu), c.455G>A, (p.Cys152Tyr), c.3462_3463del, (p.Cys1154_Glu1155delinsTer), c.3037C>T, (p.Gln1013Ter), c.2673C>A, (p.Cys891Ter), c.2230C>T, (p.Arg744Ter), c.3676G>T, (p.Gly1226Ter), c.2842+5G>A, c.2842T>C, (p.Cys948Arg), c.3988del, (p.Glu1330fs), c.2506C>A, (p.Pro836Thr), c.2291G>A, (p.Arg764His), c.1576C>T, (p.Arg526Ter), c.613_619del, (p.Ile205fs), c.3320T>C, (p.Leu1107Pro), c.2688T>A, (p.Cys896Ter), c.2555T>C, (p.Ile852Thr), c.2222T>C, (p.Met741Thr), c.1148G>A, (p.Cys383Tyr), c.2843G>A, (p.Cys948Tyr), c.4121_4130del, (p.Ala1374fs), c.3307G>A, (p.Gly1103Arg), c.484G>A, (p.Val162Met), c.2401A>T, (p.Lys801Ter), c.2234C>T, (p.Thr745Met), c.2290C>T, (p.Arg764Cys), c.3122T>C and (p.Met1041Thr).

Preferably, the mutation of the CRB1 gene of the model or the model carrier is an Rd8 mutation.

Preferably, the mutation is a homozygous mutation or a heterozygous mutation.

Preferably, the mutation of the gene as described above is innately present in vivo in the model or the model carrier or the mutation is acquired due to genetic recombination operations.

Preferably, a humanized CRB1 gene or a human CRB1 gene is present in vivo in the model or the model carrier, and its endogenous CRB1 gene is deleted or not expressed.

Preferably, the non-human animal has a colonic epithelial barrier defect and/or associated inflammation of a colonic wall.

In one specific embodiment, an Occludin protein is significantly deleted in vivo in the model. In one specific embodiment, the Occludin protein is significantly deleted and Claudin1 expression is not significant in vivo in the model.

The microorganism is one or a combination of two or more of bacteria, archeobacteria, protists, fungi, or viruses, preferably, the microorganism is the bacteria, and the bacteria are selected from: one or two or more of *Anearostipes, Bifidobacterium, Megamonas, Nitrosomonas, Oscillibacter, Tatumella, Thiobacillus* sp., *Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, Streptomyces, Anaeroplasma,* and *Coprococcus.*

Specifically, the bacteria are selected from: one or two or more of *Anearostipes hadrus, Bifidobacterium pseudocatenulatum, Nitrosomonas* sp.Is79A3, *Oscillibacter valericigenes, Tatumella* sp.TA1, *Megamonas funiformis, Thiobacillus denitrificans, Clostridium tetani, Clostridium perfringens, Clostridium botulinum, Acinetobacter calcoaceticus, Acinetobacter lwoffi, Acinetobacter baumannii, Acinetobacter haemolyticus, Acinetobacter junii, Acinetobacter johnsonii, Streptococcus pyogenes, Streptococcus haemolyticus, Fibrobacter succinogenes, intestinal Fibrobacter, Porphyromonas asacharolytica, Porphyromonas endodontalis, Porphyromonas gingivalis, Campylobacter jejuni, Campylobacter coli, Campylobacter laridis, Campylobacter upsaliensis, Campylobacter concisus, Campylobacter fetus, Actinomyces israelii, Actinomyces naeslundii, Actinomyces odontolyticus, Actinomyces viscosus, Actinomyces neuii, Escherichia coli, Escherichia blattae, Escherichia fergusonii, Escherichia hermannii, Escherichia vulneris, Tissierella praeacuta, Klebsiella pneumoniae, Klebsiella ozaenae, Azospirillum brasilense, Achromobacter, Thiobacillus denitrificans, Thiobacillus ferrooxidans, Thiobacillus thiooxidans, Thiobacillus neapolitanus, Burkholderia, Mycobacterium marinum, Treponema pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella boydii, Shigella sonnei, Frankia, Coprococcus eutactus, Streptomyces albus, Pseudomonas mendocina, Micrococcus sedentarius, alicycline denitrifying bacteria, Achromobacter xylosoxidans, Sphingomonas, Mycobacterium abscessus, Arthrobacter aurescens, Prevotella, Sinorhizobium meliloti, acidic yeast, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Haemophilus vaginalis, bee Enterococcus faecium, Cytophaga hutchinsonii, Bacillus licheniformis, Xanthomonas oryzae pv. oyzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia, Lactobacillus delbrueckii, Meiothermus silvanus (D), Escherichia coli, Micrococcus luteus, Bacillus subtilis, Corynebacterium aurimucosum,* and *Finegoldia magna.*

Preferably, a method of the infecting includes contacting the microorganism with a to-be-infected site of the model carrier directly or indirectly, wherein the indirect contact means that a blood-retinal barrier is present between the microorganism and the to-be-infected site, preferably the blood-retinal barrier is an outer blood-retinal barrier or an inner blood-retinal barrier.

In one specific embodiment, the method of the infecting includes causing intestinal bacteria to infect eyes via peripheral blood. Specifically, an intestinal epithelial barrier of a non-human animal model is significantly damaged, causing intestinal bacteria to enter the peripheral blood. In one model, the retinal barrier is also significantly damaged such that intestinal bacteria enter the peripheral blood to infect a retina; in one specific embodiment, the non-human animal model has a mutation in the CRB1 gene; and in one specific embodiment, the mutation of the CRB1 gene is an Rd8 mutation.

Further, the present invention provides a method for preparing an ocular inflammation model, including infecting a non-human animal with a microorganism.

Preferably, the ocular inflammation is caused by an intestinal flora or the same flora as the intestinal flora.

The non-human animal is the animal as described above.

The microorganism is the microorganism as described above.

Further, the present invention provides a method for preparing a retinal degenerative disease model, including infecting a non-human animal suffering from retinal degeneration with a microorganism.

Preferably, the retinal degenerative disease is the retinal degeneration as described above.

The non-human animal is the animal as described above.

The microorganism is the microorganism as described above.

Further, the present invention provides a model carrier having ocular inflammation caused by infection with a microorganism.

Preferably, the microorganism is derived from intestinal bacteria of the same individual or is the same as the intestinal bacteria of the individual.

Preferably, the eye disease includes retinal degeneration; more preferably, the retinal degeneration is progressive retinal degeneration.

Preferably, the retinal degeneration is inherited retinal degeneration (IRD).

Preferably, the retinal degeneration is a disease of a model animal itself or is caused by genetic manipulation in the model animal.

Preferably, the eye disease includes LCA, RP, arRP, EORD, EORP, PPRPE, rettelangiectasia and/or choroideremia like fundus.

Preferably, the eye disease includes ocular inflammation, e.g., uveitis, glaucoma, and age-related macular degeneration (AMD), vitritis, choroiditis, retinitis, retinal vasculitis, optic neuritis, as well as uveitis, Behcet's disease, Vogt-Koyanagi-Harada syndrome, uveitis, retinopathy, sympathetic ophthalmia, cataract, conjunctivitis, glaucoma, and the like.

Preferably, the model is a non-human animal, preferably a monkey, a dog, a chimpanzee, a rat, and a mouse.

Preferably, the model carrier is a cell, a tissue or an organ, and the cell, the tissue or the organ is derived from a human or a non-human animal.

Preferably, the cell is a primary cell or a cell line.

Preferably, the tissue is an ocular tissue and the organ is an ocular organ.

Preferably, the tissue or the organ is a regenerative tissue or organ.

Preferably, the model has a disease-causing mutation in a gene.

Preferably, the gene with the disease-causing mutation is a gene related to maintaining a structure of a retinal barrier, the retinal barrier being an outer blood-retinal barrier and/or an inner blood-retinal barrier.

Preferably, one or two or more of the following genes in the model carrier are mutated: one or a combination of two or more of ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

In one specific embodiment, an eye disease-related gene with a disease-causing mutation in the model carrier includes the CRB1 gene.

Preferably, a mutation of the CRB1 gene of the model carrier includes one or two or more of the following mutations: c.107C>G, c.111delT, c.135C>G, c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G>A, c.3103C>T, c.4082G> A, c.4060G>A, c.866C>T, c.1463T>C, c.2035C>G, c.2306_2307GC>AG, c.2306G>A, c.2714G>A, c.2875G>A and c.3992G>A.

Further preferably, the mutation of the CRB1 gene of the model or the model carrier includes one or two or more of the following mutations: c.4006-1G>T, c.3686G>C, (p.Cys1229Ser), c.2842+1delinsAA, c.4060G>A, (p.Ala1354Thr), c.3991C>T, (p.Arg1331Cys), c.3014A>T, (p.Asp1005Val), c.4005+1G>A, c.2680_2684del, (p.Asn894fs), c.1733T>A, (p.Val578Glu), c.455G>A, (p.Cys152Tyr), c.3462_3463del, (p.Cys1154_Glu1155delinsTer), c.3037C>T, (p.Gln1013Ter), c.2673C>A, (p.Cys891Ter), c.2230C>T, (p.Arg744Ter), c.3676G>T, (p.Gly1226Ter), c.2842+5G>A, c.2842T>C, (p.Cys948Arg), c.3988del, (p.Glu1330fs), c.2506C>A, (p.Pro836Thr), c.2291G>A, (p.Arg764His), c.1576C>T, (p.Arg526Ter), c.613_619del, (p.Ile205fs), c.3320T>C, (p.Leu1107Pro), c.2688T>A, (p.Cys896Ter), c.2555T>C, (p.Ile852Thr), c.2222T>C, (p.Met741Thr), c.1148G>A, (p.Cys383Tyr), c.2843G>A, (p.Cys948Tyr), c.4121_4130del, (p.Ala1374fs), c.3307G>A, (p.Gly1103Arg), c.484G>A, (p.Va1162Met), c.2401A>T, (p.Lys801Ter), c.2234C>T, (p.Thr745Met), c.2290C>T, (p.Arg764Cys), c.3122T>C and (p.Met1041Thr).

Preferably, the mutation of the CRB1 gene of the model carrier is an Rd8 mutation.

Preferably, the mutation is a homozygous mutation or a heterozygous mutation.

Preferably, the mutation of the gene as described above is innately present in vivo in the model carrier or the mutation is acquired due to genetic recombination operations.

Preferably, a humanized CRB1 gene or a human CRB1 gene is present in vivo in the model carrier, and its endogenous CRB1 gene is deleted or not expressed.

Preferably, the non-human animal has a colonic epithelial barrier defect and/or associated inflammation of a colonic wall.

In one specific embodiment, an Occludin protein is significantly deleted in vivo in the model. In one specific embodiment, the Occludin protein is significantly deleted and Claudin1 expression is not significant in vivo in the model.

The microorganism is one or a combination of two or more of bacteria, archeobacteria, protists, fungi, or viruses, preferably, the microorganism is the bacteria, and the bacteria are selected from: one or two or more of *Anearostipes, Bifidobacterium, Megamonas, Nitrosomonas, Oscillibacter, Tatumella, Thiobacillus* sp., *Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, Streptomyces, Anaeroplasma,* and *Coprococcus.*

Specifically, the bacteria are selected from: one or two or more of *Anearostipes hadrus, Bifidobacterium pseudocatenulatum, Nitrosomonas* sp.Is79A3, *Oscillibacter valericigenes, Tatumella* sp.TA1, *Megamonas funiformis, Thiobacillus denitrificans, Clostridium tetani, Clostridium perfringens, Clostridium botulinum, Acinetobacter calcoaceticus, Acinetobacter lwoffi, Acinetobacter baumannii, Acinetobacter haemolyticus, Acinetobacter junii, Acinetobacter johnsonii, Streptococcus pyogenes, Streptococcus haemolyticus, Fibrobacter succinogenes, intestinal Fibrobacter, Porphyromonas asacharolytica, Porphyromonas endodontalis, Porphyromonas gingivalis, Campylobacter jejuni, Campylobacter coli, Campylobacter laridis, Campylobacter upsaliensis, Campylobacter concisus, Campylobacter fetus, Actinomyces israelii, Actinomyces naeslundii, Actinomyces odontolyticus, Actinomyces viscosus, Actinomyces neuii, Escherichia coli, Escherichia blattae, Escherichia fergusonii, Escherichia hermannii, Escherichia vulneris, Tissierella praeacuta, Klebsiella pneumoniae, Klebsiella ozaenae, Azospirillum brasilense, Achromobacter, Thiobacillus denitrificans, Thiobacillus ferrooxidans, Thiobacillus thiooxidans, Thiobacillus neapolitanus, Burkholderia, Mycobacterium marinum, Treponema pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella boydii, Shigella sonnei, Frankia, Coprococcus eutactus, Streptomyces albus, Pseudomonas mendocina, Micrococcus sedentarius, alicycline denitrifying bacteria, Achromobacter xylosoxidans, Sphingomonas, Mycobacterium abscessus, Arthrobacter aurescens, Prevotella, Sinorhizobium meliloti, acidic yeast, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Haemophilus vaginalis, bee Enterococcus faecium, Cytophaga hutchinsonii, Bacillus licheniformis, Xanthomonas oryzae pv. oyzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia, Lactobacillus delbrueckii, Meiothermus silvanus (D), Escherichia coli, Micrococcus luteus, Bacillus subtilis, Corynebacterium aurimucosum,* and *Finegoldia magna.*

Preferably, a method of the infecting includes contacting the microorganism with a to-be-infected site of the model carrier directly or indirectly, wherein the indirect contact means that a blood-retinal barrier is present between the microorganism and the to-be-infected site, preferably the blood-retinal barrier is an outer blood-retinal barrier or an inner blood-retinal barrier.

In one specific embodiment, the method of the infecting includes causing intestinal bacteria to infect eyes via peripheral blood. Specifically, an intestinal epithelial barrier of a non-human animal model is significantly damaged, causing intestinal bacteria to enter the peripheral blood. In one model, the retinal barrier is also significantly damaged such that intestinal bacteria enter the peripheral blood to infect a retina; in one specific embodiment, the non-human animal model has a mutation in the CRB1 gene; and in one specific embodiment, the mutation of the CRB1 gene is an Rd8 mutation.

The model carrier having inflammation is derived from a disease model prepared by the method as described above, or is obtained by infecting an ocular cell, tissue or organ derived from the non-human animal as described above with a microorganism.

The present invention provides application of the method as described above in evaluating the efficacy of targeted treatment of an eye disease, the eye disease including the eye disease as described above.

In one specific embodiment, targeted treatment for the gene mutations as described above is performed on the model or the model carrier, the model or the model carrier after targeted treatment and without targeted treatment is divided into two groups, and eye disease models are respectively built according to the method as described above. If, in contrast, the group after targeted treatment cannot be successfully modeled, it is indicated that the targeted treatment has achieved beneficial effects.

In one specific embodiment, the targeted treatment targets one or a combination of two or more of the following genes: ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

In one specific embodiment, the targeted treatment targets one or two or more of the following mutations of the CRB1 gene: c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G>A, c.3103C>T, c.4082G> A, c.4060G>A, c.866C>T, c.1463T>C, c.2035C>G, c.2306_2307GC>AG, c.2306G>A, c.2714G>A, c.2875G>A and c.3992G>A.

In one specific embodiment, a drug for the targeted treatment includes modified cells, modified proteins, RNA targeting the genes as described above or targeting sites of the mutations as described above, and/or DNA targeting the genes as described above or targeting sites of the mutations as described above.

The present invention provides application of a disease model prepared by the method as described above in research related to an eye disease. The eye disease includes the eye disease as described above. The research includes interaction between diseases related to inherited retinal degeneration and an intestinal flora, etc.

In one specific embodiment, the disease model is infected with one intestinal bacterium, the efficacy of administration and non-administration is observed when a disease-causing mutation carried by the disease model is treated by cells, and a drug is a small molecule drug, preferably a broad-spectrum antibiotic or an antibiotic against the infected bacterium. In one specific embodiment, the disease model is infected with one intestinal bacterium, the efficacy of administration and non-administration is observed when a disease-causing mutation carried by the disease model is treated by RNA, and the drug is a small molecule drug, preferably a broad-spectrum antibiotic or an antibiotic against the infected bacterium. In one specific embodiment, the disease model is infected with two or more intestinal bacteria, the efficacy of administration and non-administration is observed when a disease-causing mutation carried by the disease model is treated by cells, and the drug is a small molecule drug, preferably a broad-spectrum antibiotic or an antibiotic against the infected bacteria. In one specific embodiment, the disease model is infected with two intestinal bacteria, the efficacy of administration and non-administration is observed when a disease-causing mutation carried by the disease model is treated by RNA, and the drug is a small molecule drug, preferably a broad-spectrum antibiotic or an antibiotic against the infected bacteria.

Preferably, the disease-causing mutation occurs in the genes as described above, or the disease-causing mutation is a mutation in the CRB1 gene as described above.

The present invention provides application of a disease model carrier prepared by the method as described above in research related to an eye disease. The eye disease includes the eye disease as described above. The research includes synergy between diseases related to inherited retinal degeneration and an intestinal flora, etc.

In one specific embodiment, the disease model carrier is infected with one intestinal bacterium, the efficacy of administration and non-administration is observed when a disease-causing mutation carried by the disease model carrier is treated by cells, and a drug is a small molecule drug, preferably a broad-spectrum antibiotic or an antibiotic against the infected bacterium. In one specific embodiment, the disease model carrier is infected with one intestinal bacterium, the efficacy of administration and non-administration is observed when a disease-causing mutation carried by the disease model carrier is treated by RNA, and the drug is a small molecule drug, preferably a broad-spectrum antibiotic or an antibiotic against the infected bacterium. In one specific embodiment, the disease model carrier is infected with two or more intestinal bacteria, the efficacy of administration and non-administration is observed when a disease-causing mutation carried by the disease model carrier is treated by cells, and the drug is a small molecule drug, preferably a broad-spectrum antibiotic or an antibiotic against the infected bacteria. In one specific embodiment, the disease model carrier is infected with two intestinal bacteria, the efficacy of administration and non-administration is observed when a disease-causing mutation carried by the disease model carrier is treated by RNA, and the drug is a small molecule drug, preferably a broad-spectrum antibiotic or an antibiotic against the infected bacteria.

Preferably, the disease-causing mutation occurs in the genes as described above, or the disease-causing mutation is a mutation in the CRB1 gene as described above.

The present invention provides application of the disease model or the disease model carrier in screening a drug related to an eye disease. The drug includes one or a combination of two or more of a small molecule drug, a chemical drug, a high molecular drug, a biological drug or a natural drug (e.g., a traditional Chinese medicine or a traditional Chinese medicine extract), a cellular drug, an RNA drug, and a DNA drug.

The eye disease includes the eye disease as described above.

Preferably, a small molecule compound is an antibiotic, and the antibiotic is a broad-spectrum antibiotic drug generally well known to those skilled in the art.

Preferably, the small molecule compound is a non-broad-spectrum antibiotic targeted to specific bacteria.

Preferably, the cells include one or a combination of two or more of modified immune cells, such as T cells, B cells or stem cells.

Preferably, the RNA includes mRNA, siRNA, sgRNA, miRNA, ASO and/or a replicon RNA.

In one specific embodiment, targeted treatment is performed on the disease model or the disease model carrier, a drug is administered or not administered simultaneously to the disease model or the disease model carrier with targeted treatment and the disease model or the disease model carrier without targeted treatment, the progression of inflammation in the groups is observed, and the efficacy of the drug for targeted treatment is evaluated.

In one specific embodiment, targeted treatment is performed on the disease model or the disease model carrier, a drug is administered or not administered simultaneously to the disease model or the disease model carrier with targeted treatment and the disease model or the disease model carrier without targeted treatment, the progression of inflammation in the groups is observed, and the efficacy of the small molecule drug is evaluated.

The chemical drug according to the present invention is selected from one or two or more of β-lactam antibiotics: including penicillins, cephalosporins, thienamycins, monobactams, β-lactamase inhibitors, methoxypenicillins and the like; aminoglycoside antibiotics: including streptomycin, gentamicin, kanamycin, tobramycin, amikacin, neomycin, ribostamycin, micronomycin, astromicin and the like; tetracycline antibiotics:
including tetracycline, oxytetracycline, chlortetracycline and doxycycline, and the like;
chloramphenicol antibiotics: including chloramphenicol, thiamphenicol and the like;
macrolide antibiotics: including erythromycin, leucomycin, erythromycin estolate, acetylspiramycin, midecamycin, josamycin, azithromycin, and the like; glycopeptide antibiotics: vancomycin, norvancomycin, teicoplanin and the like; quinolone antibiotics:
   including norfloxacin, ofloxacin, ciprofloxacin, pefloxacin, and gatifloxacin; nitroimidazole antibiotics: including metronidazole, tinidazole, omidazole, and the like; rifamycin antibiotics: including rifampicin and the like; echinocandin antibiotics; polyene antibiotics;
   pyrimidine antibiotics; allylamine antibiotics; azole antibiotics; and other antibiotics:
      fosfomycin, capreomycin, cycloserine, lincomycin, clindamycin, mitomycin, dactinomycin, bleomycin, doxorubicin, isoniazid, pyrazinamide, cyclosporine and the like.

The biological drug according to the present invention is an antimicrobial peptide selected from one or two or more of insect antimicrobial peptides, e.g., lepidopteran antimicrobial peptides, Dipteran antimicrobial peptides, Coleopteran antimicrobial peptides, Odonata antimicrobial peptides, Hymenopteran antimicrobial peptides, moricin, and the like; mammalian antimicrobial peptides, e.g., porcine antimicrobial peptides, ovine antimicrobial peptides, bovine antimicrobial peptides, human antimicrobial peptides, etc.; amphibian antimicrobial peptides: magainin and the like; antimicrobial peptides derived from fish, mollusks, and crustaceans: pardaxin, parasin, myticin, penaeidins, etc.; plant antimicrobial peptides: Thi-onins, etc., and bacterial antimicrobial peptides: bacitracin, gramicidin, polymyxin and nisin, etc.

The natural drug according to the present invention is selected from one or two or more of radix astragali, rhizoma polygonati, radix angelicae sinensis, notoginseng, rhizoma imperatae, charred radix et rhizoma rhei, radix curcumae, thunberg fritillary bulb, coix seeds, rhizoma pinellinae praeparata, calcined ancient ink, radix salviae miltiorrhizae, radix lithospermi, radix isatidis, herba houttuyniae, honeysuckle, rhizoma coptidis, radix scutellariae, dandelion, herba portulacae, hawthorn, folium isatidis, fructus forsythiae, herba artemisiae scopariae, herba andrographitis, radix bupleuri, herba euphorbiae humifusae, radix stemonae, garlic, cortex phellodendri, cortex eucommiae, cortex fraxini, fructus cnidii, rhizoma coptidis, Chinese gall, herba violae, smoked plum, licorice, pomegranate rind, fructus schisandrae, Chinese honeylocust spine, fructus chebulae, radix sophorae flavescentis, cortex pseudolaricis, herba epimedii, sweet wormwood or an extract thereof.

The drug of the present invention may be an oral drug, an injectable drug or a topical drug, and the topical drug includes a mucosally administered drug, preferably an ophthalmically administered drug.

A dosage form of the drug of the present invention may be a solution, a tablet, a pill, a capsule, an injection solution, a powder, a powder for injection, a patch, a paint, or a preparation for mucosal administration, and the preparation for mucosal administration is preferably an eye drop, an eye ointment, an eye spray preparation or the like.

### BRIEF DESCRIPTION OF THE DRAWINGS/FIGURES

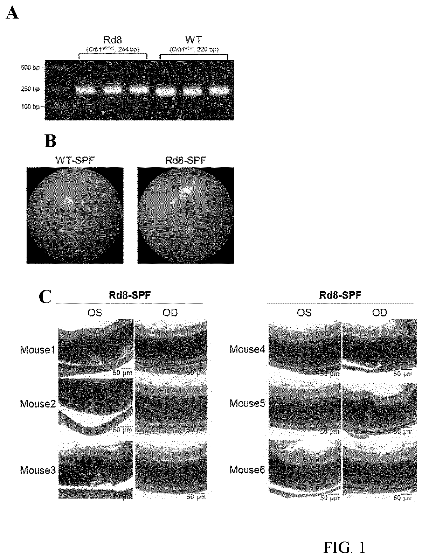

FIG. 1: Genotype and phenotype of Crb1^{rd8/rd8} (rd8)-SPF mice. Wherein FIG. 1A shows gel electropherogram of genotype of Crb1^{rd8/rd8} (rd8) and Crb1^{wt/wt} (C57BL/J, abbreviated as wt) mice, FIG. 1B shows representative fundus images of Rd8-SPF and WT-SPF mice, with typical white spots appearing in an inferior nasal quadrant of retinas of the Rd8-SPF mice, and FIG. 1C shows H&E staining for detecting pathological changes in both eyes of Rd8-SPF mice at E18.

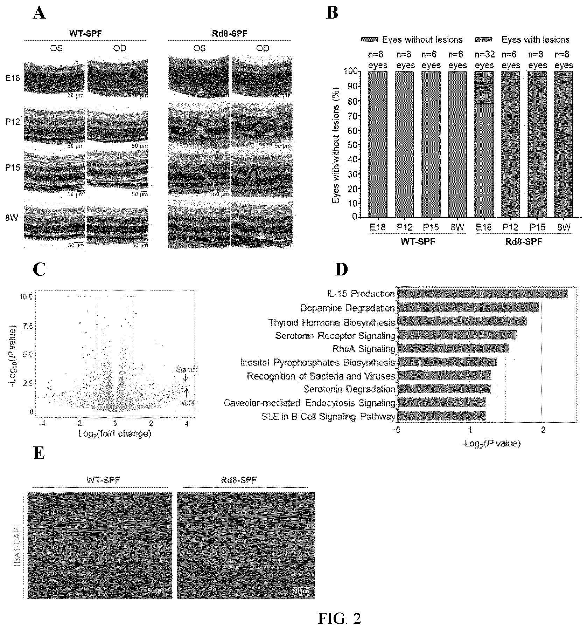

FIG. 2: Potential mechanism of retinal abnormality and retinopathy in Rd8-SPF mice. Wherein FIG. 2A shows pathologic changes in both eyes of Rd8-SPF and WT-SPF mice at E18, P12 (before eye opening), P15 (after eye opening) and 8 weeks old (8W) detected by H&E staining, Rd8 mice exhibit typical retinal abnormalities including progressive retinal dysplasia (folds and pseudowreaths) and degeneration, while WT mice exhibit a normal retinal structure; FIG. 2B shows statistical data of eyes with/without lesions in Rd8-SPF and WT-SPF mice at E18, P12, P15 and 8W, typical lesions can be seen in all retinas in Rd8 mice at P12, P15 and 8W, but none in WT-SPF mice, WT-SPF: n=6 in each group at E18, P12, P15 and 8W; Rd8-SPF: n=32 at E18, n=6 at P12, and n=8 at P15; and FIG. 2C shows volcano plot analysis of DEGs in a superior retina (without lesions) and an inferior retina (with lesions) of Rd8 mice. Slamf1 (a signaling lymphocytic activation molecule family member 1) and Ncf4 (a neutrophil cytosolic factor) are two genes with the highest fold proliferation in retinopathy in Rd8-SPF mice; FIG. 2D shows IPA functional analysis of DEGs; and FIG. 2E shows immunofluorescence staining showing that there are abundant IBA1+microglia (red) in a retinopathy area of Rd8 mice.

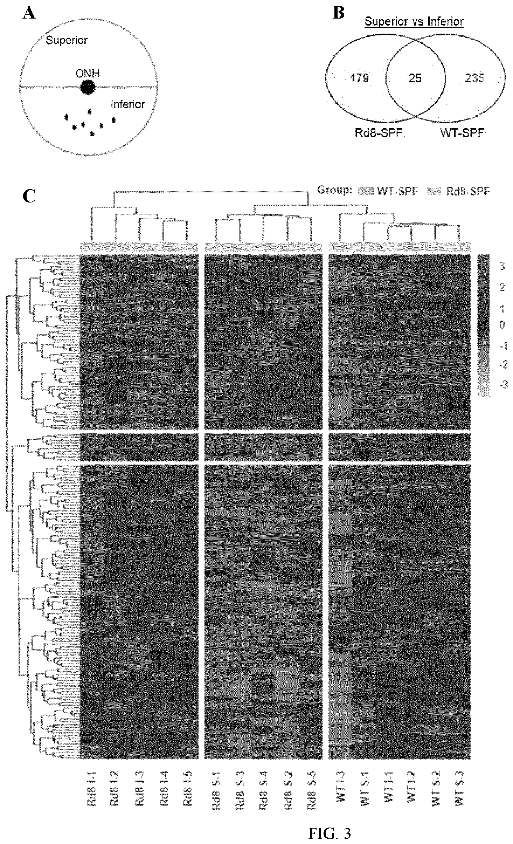

FIG. 3: RNA sequence analysis and comparison of gene expression profiles in a superior retina and an inferior retina of Rd8-SPF mice. Wherein FIG. 3A is a graphical representation of superior (without lesions) and inferior (with lesions) regions of Rd8-SPF mice; FIG. 3B shows that RNA-seq analysis shows there is at least a 2-fold difference in expression in a superior retina and an inferior retina of Rd8-SPF mice (P<0.05); and FIG. 3C shows an expression pattern of 179 DEGs in a superior retina and an inferior retina of Rd8-SPF and WT-SPF mice.

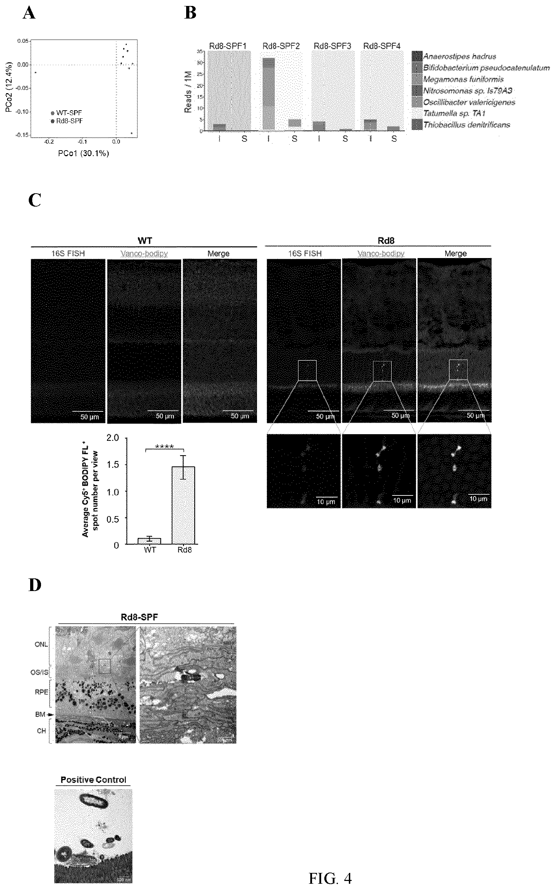

FIG. 4: Identification of bacteria within retinopathy in Rd8-SPF mice. Wherein FIG. 4A is a principal coordinate analysis (PCoA) showing that the bacterial composition in a retinal tissue of Rd8 mice is significantly different from that of WT mice; FIG. 4B: Identification of microbial species composition in superior (S) and inferior (I) tissues of retinas of Rd8 mice by metagenomic sequencing (n=4); FIG. 4C shows detection of bacterial 16S rDNA and vanco-bodipy in retinas of Rd8-SPF and WT-SPF mice (4 weeks old) by fluorescence in situ hybridization; and FIG. 4D: Observation of bacterial distribution in retinopathy of Rd8 mice by a transmission electron microscope (TEM), a positive control being coliform;

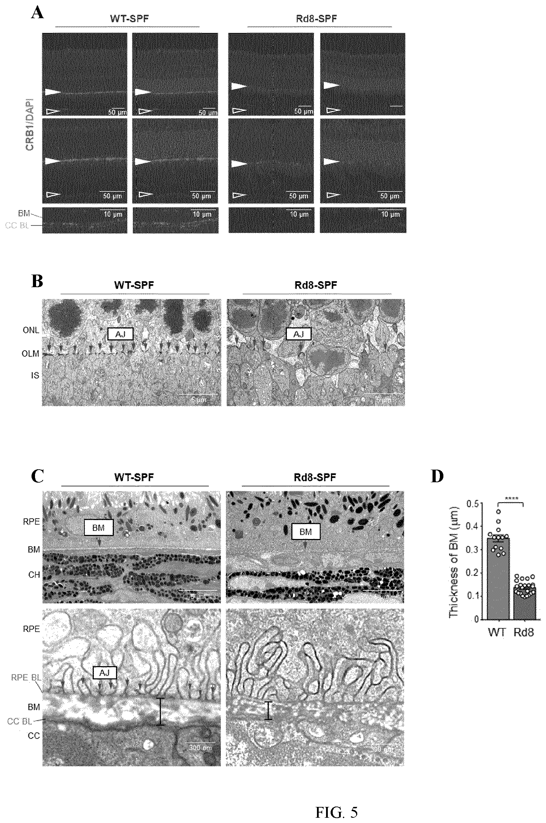

FIG. 5: Destruction of adherens junctions in retinas of Rd8-SPF mice. Wherein FIG. 5A shows immunofluorescence staining of CRB1 (red) in retinas of Rd8-SPF and WT-SPF mice, BM refers to a Bruch's membrane, CC refers to chorionic capillaries, and BL refers to a basal lamina; FIG. 5B shows a transmission electron microscopic observation of adherens junctions of outer limiting membranes of retinas of Rd8-SPF and WT-SPF mice, red arrows refer to adherens junctions, AJ refers to adherens junction, OLM refers to an outer limiting membrane, ONL refers to an outer nuclear layer, and IS refers to an inner segment; FIG. 5C shows lysis of adherens junctions in a basal layer of a retinal pigment epithelium (RPE), distortion of a basal layer of chorionic capillaries, and disturbances of a collagen layer in Rd8-SPF mice, CC refers to chorionic capillaries, CH refers to choroid, and BL refers to a basal lamina; and FIG. 5D shows statistical analysis of Bruch's membrane thickness in Rd8-SPF and WT-SPF mice, ****P<0.0001.

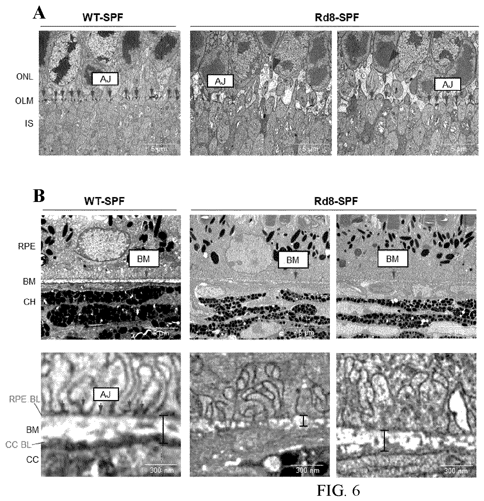

FIG. 6A: Transmission electron microscopy (TEM) shows cleavage of adherens junction (AJ) of an outer limiting membrane (OLM) and an outer nuclear layer (ONL) of Rd8-SPF mice.
FIG. 6B: Transmission electron microscopy (TEM) shows the thickness of a Bruch's membrane in Rd8-SPF mice and WT-SPF mice.

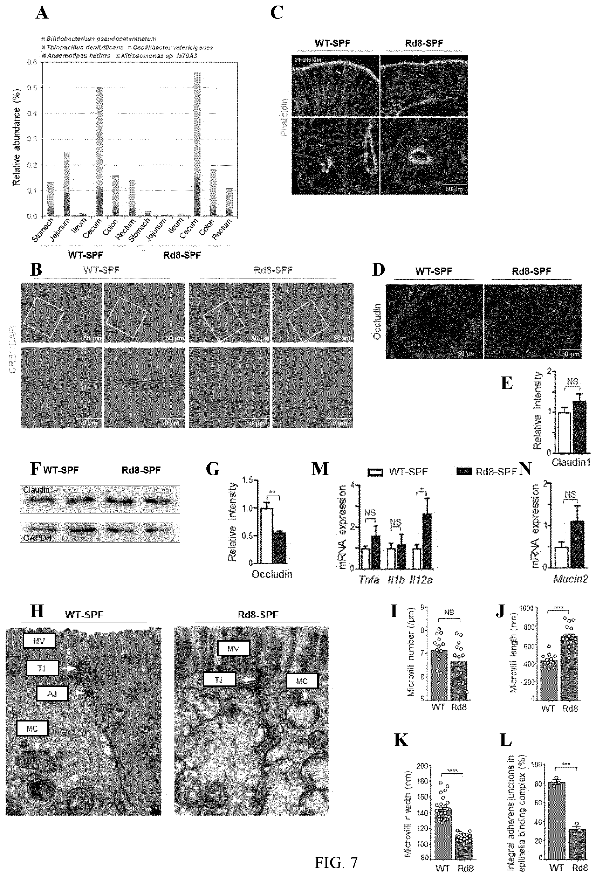

FIG. 7: Colonic epithelial barrier defects and associated inflammation thereof in Rd8-SPF mice. Wherein FIG. 7A shows the relative abundance of bacteria in different parts of a gastrointestinal tract of Rd8 and WT mice (n=5); FIGS. 7B-D show immunofluorescence staining of a CRB 1 protein (B), phalloidin (C) and occludin (D) in colonic intestinal cells of Rd8-SPF and WT-SPF mice; FIG. 7E shows statistical analysis of the relative intensity of occludin in Rd8-SPF and WT-SPF mice; FIG. 7F shows the expression of Claudin1 in colonic intestinal epithelial cells of Rd8-SPF and WT-SPF mice detected by Western blot; FIG. 7G show statistical analysis of the relative intensity of Claud in 1 in Rd8-SPF and WT-SPF mice; FIG. 7H shows adherent and tight junctions between colon epithelia of Rd8-SPF and WT-SPF mice observed under a transmission electron microscope; FIGS. 7I-L show statistical analysis of the number of epithelial microvilli (I), a microvilli length (J), a microvilli width (K) and the total number of integral adherens junctions (L) in Rd8-SPF and WT-SPF mice; and FIGS. 7M-N show a comparison of mRNA expression levels of Tnfa, Il1b, and Il12a (M) and Mucin2 (N) in Rd8-SPF and WT-SPF mice.

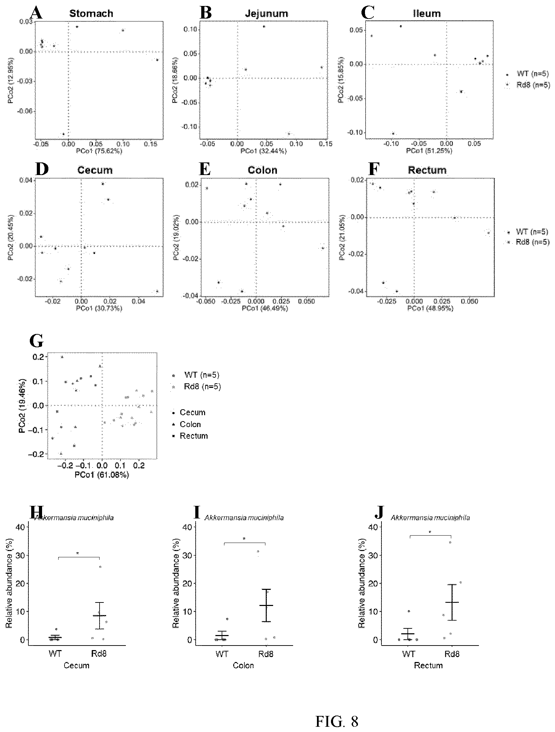

FIG. 8: Microbial species composition of flora in different parts of a gastrointestinal tract of Rd8-SPF mice. Wherein FIGS. 8A-F show principal coordinate analysis (PCoA) of the microbial species composition in gastrointestinal samples of WT (n=5) and Rd8 (n=5) mice based on a Bray-Curtis distance, including a stomach (FIG. 8A), a jejunum (FIG. 8B), an ileum (FIG. 8C), a cecum (FIG. 8D), a colon (FIG. 8E) and a rectum (FIG. 8F); FIG. 8G shows the microbial composition of a lower digestive tract (a cecum, a colon and a rectum) of Rd8 and WT-SPF mice distinguished by PCoA; and FIGS. 8H-J show a comparison of the relative abundance of Akkermansia muciniphila in the cecum (H), the colon (I) and the rectum (J) of Rd8 and WT mice. Data are expressed as mean±SEM, *P<0.05.

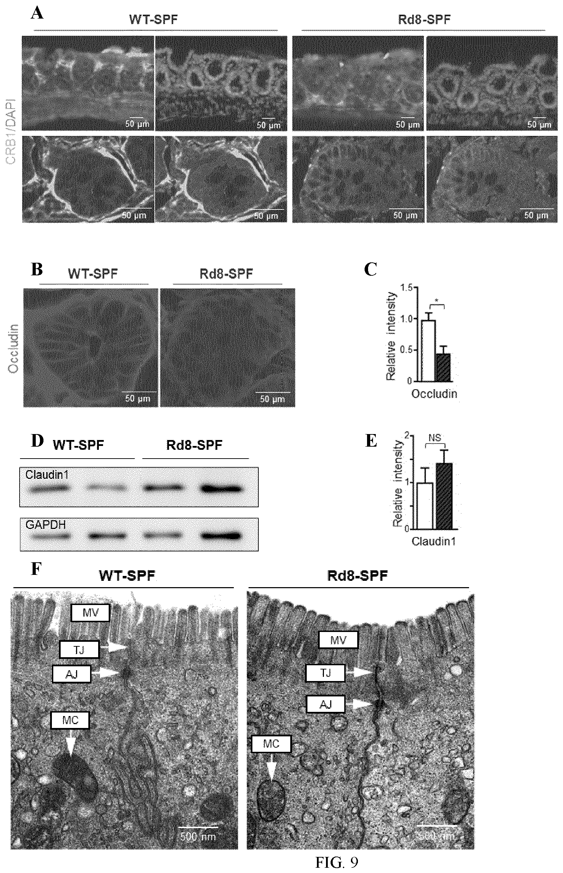

FIG. 9: Tight junctions and adherens junctions in ceca of Rd8 mice. Wherein FIG. 9A shows immunofluorescence staining of a CRB 1 protein (green) in cecal intestinal cells of Rd8-SPF and WT-SPF mice; FIG. 9B shows immunofluorescence staining of occludin (red) in cecal intestinal cells of Rd8-SPF and WT-SPF mice; FIG. 9C shows statistical analysis of the relative intensity of occludin in Rd8-SPF and WT-SPF mice, *P<0.05; FIG. 9D shows the expression of Claud in 1 in ceca of Rd8-SPF and WT-SPF mice detected by Western blot; FIG. 9E shows statistical analysis of the relative intensity of Claud in 1 in Rd8-SPF and WT-SPF mice, and NS represents unimportance; and FIG. 9F shows transmission electron microscopy showing normal tight and adherens junctions at a cecal epithelial barrier, MV refers to microvesicles, and MC refers to mitochondria.

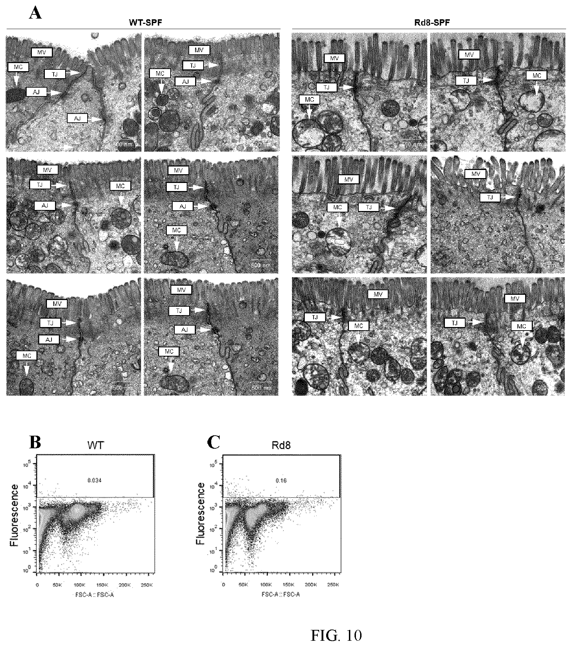

FIG. 10: FIG. 10A shows the tight junction (TJ) and adherens junction (AJ) between colon epithelia of Rd8-SPF and WT-SPF mice observed under a transmission electron microscope, MV refers to microvesicles and MC refers to mitochondria; and FIG. 10B shows the frequency of fluorescence+bacteria/cells in peripheral blood of Rd8 and WT mice detected by flow cytometry.

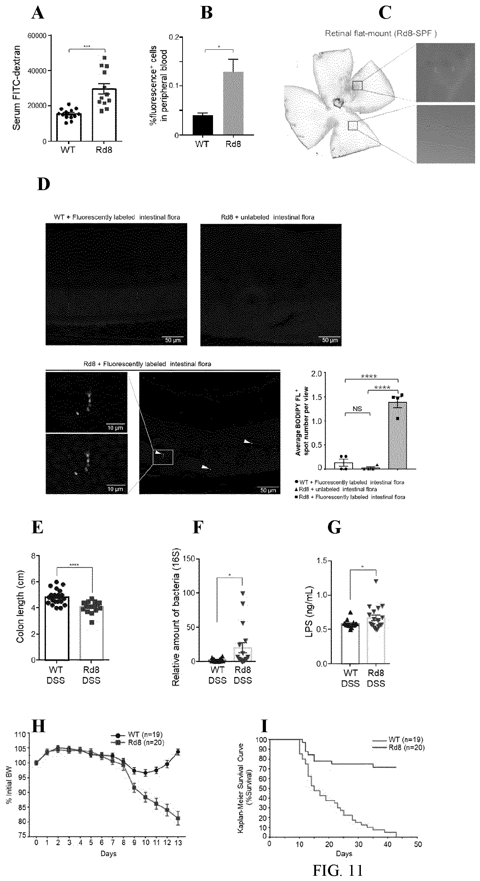

FIG. 11: Disruption of the intestinal epithelial barrier function in Rd8-SPF mice. Wherein FIG. 11A shows intestinal permeability determination with FICT-dextran showing the serum fluorescence intensity of Rd8 mice is significantly higher than that of WT mice, ***P<0.001; FIG. 11B shows a comparison of the percentage of fluorescence+bacteria/cells in peripheral blood of Rd8 and WT mice by vanco-bodipy labeled fecal microbiota transplantation, *P<0.05; FIGS. 11C-D show that vanco-bodipy+bacteria can be detected in retinopathy of Rd8 mice under a fluorescence microscope (C) and by immunofluorescence staining (D); FIGS. 11F-G show a comparison of a colon length, and serum levels of bacterial 16S rRNA (F) and lipopolysaccharide (LPS) (G) of Rd8 and WT mice after treatment with dextran sulfate sodium (DSS) for 13 days, ****P<0.0001; FIG. 11H shows a body weight of Rd8 (n=20) and WT (n=19) mice treated with 2.5% DSS monitored daily; and FIG. 11I shows a Kaplan-Meier survival curve of Rd8 (n=20) and WT (n=19) mice treated with 2.5% DSS.

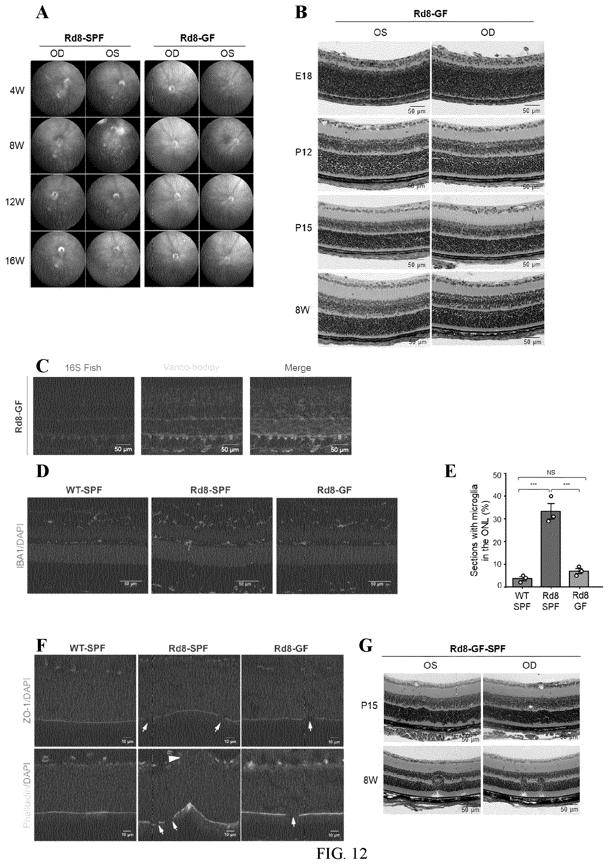

FIG. 12: Retinal phenotype of Rd8 GF and Rd8 GF SPF mice. Wherein FIG. 12A shows representative fundus images of Rd8-SPF and Rd8 GF mice at 4 weeks, 8 weeks, 12 weeks, and 16 weeks; FIG. 12B shows a histological observation of retinas in both eyes of Rd8-GF mice at E18, P12, P15 and 8W; FIG. 12C shows fluorescence in situ hybridization staining of bacterial 16S rDNA in retinas of Rd8-GF mice and vanco-bodipy; FIG. 12D shows immunofluorescence staining of IBA1 (red) in retinas of Rd8-SPF, Rd8 GF and WT-SPF mice; FIG. 12E shows the percentage of IBA1+microglia in an outer nuclear layer (ONL) of Rd8-SPF, Rd8 GF and WT-SPF mice, ***P<0.001; NS represents unimportance; FIG. 12F shows immunofluorescence staining of ZO-1 (red) and Phalloidin (green) proteins in retinas of Rd8-SPF, Rd8-GF and WT-SPF mice; and FIG. 12G shows retinopathy in Rd8-GF mice raised in a specific pathogen-free (SPF) environment after birth (Rd8-GF-SPF mice) at P15 and 8W detected by H&E staining.

### DETAILED DESCRIPTION OF THE INVENTION

The technical solutions in the examples of the present invention will be clearly and completely described below. In the examples provided below, only a modeling method in which mice with a mutation in a Crb1 gene are raised in a specific pathogen-free (SPF) environment is used, and it is determined that the retina of the model is infected with bacteria by verifying the presence of local inflammatory response in its retina and the presence of bacteria at a lesion site. Further, through the following experiments, it is verified that the bacteria are from an intestinal tract. This specific example does not exclude other modeling methods, such as raising in an environment with more complex microbial conditions, or applying microorganisms from an intestinal tract or the same microorganisms as intestinal microorganisms to ocular tissues, allowing an ocular tissue to be in direct or indirect contact with the microorganisms as described above, and the like.

Obviously, the described examples are only some but not all of the examples of the present invention. Based on the examples in the present invention, all other examples obtained by those of ordinary skill in the art without making inventive steps are within the scope of protection of the present invention.

### 1. Mice

C57BL/6N mice (Crb1^{rd8/Rd8}, designated as Rd8 mice) and C57BL/6J mice (Crb1^{wt/wt}, designated as wt mice) carrying an Rd8 mutation were purchased from Beijing Vital River Laboratory Animal Technology Co., Ltd. and maintained under specific pathogen-free (SPF) conditions in an animal facility of the Zhongshan Ophthalmic Center. An animal facility of the First Affiliated Hospital of Sun Yat-sen University was used, and germ-free (GF) RD8 mice were generated by using embryos of female RD8 mice. GF mice were kept germ-free and facility staff performed microbiological and parasite tests on fecal samples weekly to ensure the sterility of a GF unit. Mice were genotyped as described above (Mattapallil et al., 2012). Crb1 genotypes of both mouse strains were confirmed (FIG. 4A). All animal experiments were approved by the Institutional Animal Care and Use Committee of the Zhongshan Ophthalmic Center and complied with the "Statement of Association for Research in Vision and Ophthalmology (ARVO) on the Use of Animals for Ophthalmology and Vision Research".

### 2 Method

### 2.1 Histochemistry

The mice were sacrificed by cervical dislocation, enucleated and fixed with 4% paraformaldehyde (PFA) in phosphate buffered saline (PBS) at 4°C for 24 h. Samples were washed for 3 times with PBS, dehydrated in a series of alcohols, dehydrated twice in xylene, then embedded in paraffin and serially sectioned with a microtome at 10 µm (RM 223; Leica, Wetzlar, Hesse-Darmstadt, Germany). Sections were stained with hematoxylin and eosin (H&E). H&E images were obtained from Imager.Z2 (Zeiss).

Eyes were taken, placed in 4% PFA at room temperature for 5 min, then dissected and fixed with an eye mask for 45 min. A colon tissue was taken and fixed in 4% PFA at room temperature for 4h. After PBS rinsing, the isolated eye mask and the colon were permeabilized overnight with 30% sucrose for cryoprotection, embedded into an optimal cutting temperature (OCT) compound (Cat. 4583; SAKURA, USA), and stored at -80°C before sectioning. Sections were cut at 12 µm for all immunostaining purposes.

Tissue sections were blocked with 10% donkey serum/PBST (0.1% tritonx-100/PBS) for 30 minutes and then incubated with a primary antibody overnight at 4°C. After washing with PBST, the sections were incubated with a fluorochrome-conjugated secondary antibody and fixed with Fluoromount-G (Southern Biotech, Birmingham, AL, USA). The same immunohistochemical method was employed to perform Phalloidin (A12379; thermo-Fisher) staining except that secondary antibodies were missed. Apotome (Zeiss) was equipped with a Zeiss confocal microscope (Zeiss LSM880; Zeiss, Oberkochen, Germany) and Imager.Z2. Main antibodies used in this study were as follows: anti-Crb1 (PA5-66373, ThermoFisher; 1:50), anti-Iba1 (ab178846, Abeam; 1: 500), anti-ZO-1 (61-7300, ThermoFisher; 1:500), anti-Occludin (OC-3F10, Invitrogen; 1:200), and AlexaFluor 488 phalloidin (A12379, ThermoFisher; 1:500).

### 2.2 Fundus photography

The mice were anesthetized, and pupils were dilated. Hypromellose eye drops were regularly applied to keep corneas moist. Mouse fundus photographs were obtained by using a Micron IV mouse fundus camera (Phoenix Research Laboratories, Inc., Pleasanton, CA, USA).

### 2.3 RNA-seq analysis of superior retina and inferior retina

Total RNA was extracted from a superior retina and an inferior retina with a MasterPure^{™} whole DNA and RNA Purification Kit (epicentre). RNA concentration was determined with a Qbit-RNA-HS analysis kit. A sequencing library was prepared by using a VAHTS^{™} Total RNA seq (H/M/R) Library Preparation Kit (Vazyme, China) according to a standard protocol provided by a manufacturer, and sequencing was performed on an
MGISEQ 2000RS platform.

Raw reads were first evaluated for quality control by FastQC (v0.11.8) and cutadapt (v1.15). Clean reads were aligned to a mouse genome (mm10) by using HISAT2 (v2.1.0). Gene expression data were imported into a DESeq2 package of R software (v3.6.1) for differential expression analysis. Differentially expressed genes (DEGs) were imported into Ingenuity Pathway Analysis (IPA) for functional enrichment analysis.

### 2.4 Metagenomic sequencing

Retinal samples were collected, and DNA was extracted by using a MasterPure^{™} Complete DNA and RNA Purification Kit (epicentre). Contents of a stomach, a jejunum, an ileum, a cecum, a colon and a rectum were collected, and DNA was extracted by using a QIAamp PowerFecal DNA Kit (QIAGEN). After concentration measurement, DNA was subjected to sequencing library preparation by using a VAHTS^{™} MGI Universal DNA Library Preparation Kit (Vazyme, China) according to a standard protocol provided by a manufacturer. Metagenomic sequencing was performed with MGISEQ-2000RS. Raw reads were quality-filtered by Trimmomatic (v0.36) and PRINSEQ (v0.20.4). Mouse reads were deleted by using KneadData (v0.6.1) (https://bitbucket.org/biobakery/kneaddata). Non-mouse-cleared reads were mapped to a pre-built MiniKraken database by using Kraken 2 (v2.0.9). The classification results were screened with a confidence of 0.20. A negative blank control was treated together with the samples. All species present in the negative blank control group were removed.

### 2.5 Determination of in vivo intestinal permeability with fluorescein isothiocyanate (FITC)-dextran

In vivo permeability was determined by an FITC-labeled dextran method to evaluate the barrier function. Food and water were taken out overnight and 8-week-old mice were orally administered with 50 mg of FITC-labeled dextran (FD-70; Sigma-Aldrich) per 100 grams (body weight). Serum was collected 5 h after administration and the fluorescence intensity of each sample was determined (excitation, 492 nm; emission, 525 nm).

### 2.6 Flow cytometry

WT and Rd8 mice were fasted overnight and gavaged with 1×10⁹ of Escherichia coli (designed to consistently express RFP). After gavage administration for 6h, the mice were euthanized, and 400 µL of peripheral blood was gently pipetted into a tube containing 4 mL of ACK lysis buffer (Gibco, USA) and incubated at RT for 3-5 min. After centrifugation at 300×g for 5 min, cells were fixed and permeabilized (Cytofix/perm solution, BD Biosciences, USA), and then analysis was performed by flow cytometry (MACSQuant Analyzer 10, Miltenyi Biotec, Germany).

### 2.7 Tissue preparation by electron microscope

Colons and eyes were collected immediately after euthanasia and fixed in a phosphate buffered glutaraldehyde-paraformaldehyde solution at room temperature for 1h. The colons were cut into pieces of 2 mm. Anterior segments of the eyes were excised and posterior segments of the eyes were cut into pieces of 2 mm×2 mm. Dissected tissues were placed in a fresh fixative for 12h, fixed with 1% osmium tetroxide, dehydrated and embedded in epon-resin. Regions of interest were pre-screened on micrometer-thick sections by staining with toluidine blue under an optical microscope. Ultrasound images at 80 nm were then collected, and counterstaining was performed with uranium acetate and lead citrate. Ultrasonic sections were observed with a transmission electron microscope.

### 2.8 Fluorescence in situ hybridization (FISH)

The following oligonucleotide probe was used in this study: EUB338, 5'-GCTGCCTCCGTAG-GAGT-3' (Amann et al., 1990). A 5' end of the probe bears a primary amino group to which tetramethylrhodamine isothiocyanate is covalently bound. A dye oligonucleotide conjugate (100 µM) was stored at -20°C.

Pre-fixed retinal sections were rinsed for 3 times with DEPC-treated PBS. After treatment with 0.2% Triton X-100/DEPC-treated PBS, the sections were hybridized with the probe (500 nM) overnight at 37°C and the hybridized material was mounted with Fluoromount-G.

### 2.9 Detection of gene expression level in colon tissue by real-time quantitative PCR

Fresh mouse colon tissue (~1 cm) was quickly frozen in liquid nitrogen, ground and lysed with RNA extraction lysis buffer. Total RNA was then purified by using a Qiagen RNeasy Plus kit and reversely transcribed into cDNA by using a Takara PrimeScript RT kit and a gDNA eraser. qPCR was used to detect the expression level of the corresponding genes. Data were normalized to β-actin.

### 2.10 Quantitative determination of 16S rRNA gene level in plasma by qPCR

Plasma was isolated from whole blood of WT and Rd8 mice after treatment with 2.5% DSS for 13 days. Approximately 50 µL of the plasma was used to isolate total nucleic acids by using a MasterPure^{™} Complete DNA and RNA Purification Kit (epicentre, USA). Precipitated nucleic acids were dissolved in 20 µL of nuclease-free water. qPCR analysis (ChamQ-SYBR-Color-qPCR-Master-Mix, Vazyme, China) was performed by using a LightCycler 96 system (Roche, USA). Since the DNA concentration in all samples was extremely low, an equal volume of each sample was used as a template (4 µL in 20 µL). A total bacterial load was measured by using following universal 16S rRNA gene primers: 27F 5'-AGAGTTTGATCCTGGCTCAG-3', and 534R 5'-GCATTACCGCGGCTGCTGG-3'.

### 2.11 Enzyme-linked immunosorbent assay (ELISA)

Each sample was measured directly with 100 µL of plasma. The concentration of LPS in the plasma was determined with an enzyme-linked immunosorbent kit (SEB526Ge; Cloud-Clone Corp., USA).

### 2.12 Colonization of fluorophore vanco-bodipy-labeled intestinal bacteria in Rd8 mice

Fresh fecal samples were collected in a 50 ml conical tube with sterile 1×PBS and spun until homogenous. Contents were filtered with a 0.22 µm filter (Millipore) to remove fecal residues and centrifuged to obtain intestinal microbiota, and then the intestinal microbiota was incubated with vanco-bodipy at RT for 30 minutes. Intestinal bacteria labeled with vanco-bodipy were gavaged at 1×10⁸ cfu/mouse in PBS. After gavage for 24 hours, mouse retinas were taken to be sectioned for observation.

### 2.13 Experimental study of dextran sulfate sodium (DSS)-induced colitis

Intestinal inflammation in mice was induced by long-term oral administration of 2.5% DSS (MW 36000-50000d, Yeasen, China) in drinking water. Body weight was monitored daily from day 0 to day 13 and the mice were sacrificed on day 13 and a colon length was measured. For survival analysis, the mice were allowed to freely drink 2.5% DSS in their drinking water for 43 days, and a mortality status of the mice was monitored every 24 hours during the 43 days.

### 2.14 Intestinal commensal depletion

Pregnant female mice were fed with a broad-spectrum antibiotic mixture of ampicillin (A; 1 g/L; Sigma), metronidazole (M; 1 g/L), neomycin (N; 1 g/L; Sigma), and vancomycin (V; 500 mg/L) in drinking water and pups were continued to be fed after weaning. Mice in a control group were placed in a conventional apparatus on the same rack.

### 3. Examples

### Example 1 Modeling

Retinal microenvironment characteristics of Crb1^{rd8/rd8} (rd8) and Crb1^{wt/wt} (C57BL/J, designated as wt) mice were observed (FIG. 1A). The phenotype of Rd8 mice raised in a specific pathogen-free (Rd8-SPF) environment was observed, and it was found that the Rd8 mice exhibited all typical retinal manifestations, including leukoplakia in a nasal quadrant under the eye under ophthalmoscopy (FIG. 1B), as well as progressive retinal dysplasia (folds and pseudowreaths) and retinal histological degeneration (H&E staining) (FIG. 2A). The results showed that 7 of 32 Rd8 mice exhibited mild retinal dysplasia (E18) before birth (FIG. 2A and FIG. 2B). In addition, all abnormalities at E18 occurred unilaterally (FIG. 2A and FIG. 1C), while typical lesions such as retinal folds and pseudowreaths could be seen in all retinas of Rd8 mice at P12 (before eye opening), P15 (after eye opening) and 8 weeks old (8W), but none in WT-SPF mice (FIG. 2A and FIG. 2B). The data suggest that retinal abnormalities in the Rd8 mice occur as early as embryonic E18 and the intraocular environment can drive retinal phenotype under the genetic influence of Crb1 mutations.

### Example 2 Detection whether there are intralesional pathogens and related local immune responses thereof in retinas of modeled mice (Rd8-SPF)

Transcriptomic analysis was performed by using an RNA-seq technology to compare gene expression profiles of superior (without lesions) and inferior (with lesions) regions of Rd8-SPF mice (FIG. 3A). After removal of genes with differential expression patterns (2-fold change and P<0.05) between a superior retina and an inferior retina of WT-SPF mice, it was shown that 179 genes (DEG) were found to have at least 2-fold differences in expression between a superior retina and an inferior retina of the Rd8-SPF mice (P<0.05) (FIG. 3B and FIG. 2C). In the Rd8-SPF mice, most of these 179 DEGs were highly expressed in the inferior region of the lesion (FIG. 3C). Functional analysis of these DEGs by using an Ingenuity Pathway analysis (IPA) tool showed that genes involved in bacterial and viral recognition were significantly enriched in all DEGs highly expressed in retinopathy of the Rd8-SPF mice (FIG. 2D). Importantly, the first two genes with the highest fold increase in lesions, Ncf4 and Slamf1, are regulatory factors of phagocytic antibacterial responses. The data suggest the presence of intralesional pathogens and related local immune responses thereof in retinas of the Rd8 mice, and these results were combined with abundant expression of genes with pro-inflammatory functions (FIG. 2D) and infiltration of IBA1+microglia into a retinopathy area (FIG. 2E).

### Example 3 Detection whether bacteria are present in a retinopathy site of modeled mice

Metagenomic analysis was performed on retinal tissues of WT-SPF (n=5, age=4 weeks) and Rd8-SPF (n=4, age=4 weeks) mice. It was found through the analysis that the bacterial DNA content in retinas of WT and Rd8 mice was extremely low, and no viral or fungal DNA was detected after all quality control and decontamination steps. However, as shown in FIG. 4A, principal coordinate analysis (PCoA) indicated that there was a significant difference in the bacterial composition of retinas of the WT-SPF and Rd8-SPF mice. Importantly, further metagenomic analysis of a superior retina and an inferior retina of the Rd8-SPF (n=4, age=4 weeks) mice showed significant enrichment of 7 bacteria in the inferior retina, including Anearostipes hadrus, Bifidobacterium pseudocatenulatum, Megamonas funiformis, Nitrosomonas sp.Is79A3, Oscillibacter valericigenes, Tatumella sp. TA1, and Thiobacillus denitrificans (FIG. 4B). In order to directly observe the presence of bacteria in retinopathy, Rd8-SPF mice (4 weeks old) were subjected to fluorescence in situ hybridization (FISH) and vanco-bodipy staining analysis, especially staining cell walls of Gram-positive bacteria. As shown in FIG. 4C, bacteria can only be found in lesion sites, but not in normal retinal areas. The presence of bacteria within the lesion was further confirmed by using a transmission electron microscope (TEM) (FIG. 4D). The data suggest that bacteria can be found in retinopathy in the Rd8-SPF mice.

### Example 5 It is proved that a main defect in retinas of Rd8-SPF mice is an outer blood-retinal barrier

Immunofluorescence staining data confirmed that the expression of a CRB1 protein in an outer limiting membrane of retinas of Rd8 mice was reduced or absent (FIG. 5A). The data confirmed that weak expression of the CRB1 protein could be found on a Bruch's membrane (FIG. 5A). By using a transmission electron microscope, it was found that at a retinopathy site in Rd8-SPF mice, adherens junctions on the outer limiting membrane were disrupted, which was associated with outward migration of an outer nuclear layer (FIG. 5B and FIG. 6A). Transmission electron microscopy also revealed lysed adherens junctions at a retinal pigment epithelium (RPE) basement membrane and a distorted chorionic capillary basement membrane in retinas of the Rd8-SPF mice, as well as disturbances of a collagen layer between the retinal pigment epithelium (RPE) basement membrane and the chorionic capillary basement membrane, which were not found in any WT-SPF mice (FIG. 5C and FIG. 6B). This was associated with disruption of the Bruch's membrane of the Rd8-SPF mice and significant reduction in Bruch's membrane thickness (FIG. 5C, FIG. 6B, and FIG. 5D). On the other hand, transmission electron microscopy did not reveal any changes in tight junctions between retinal capillary endothelial cells and retinal pigment epithelial cells. Thus, the results suggest that the main defect in the retinas of the Rd8-SPF mice is the outer blood-retinal barrier, rather than an inner blood-retinal barrier.

### Example 6 Metagenomic analysis of microbiota in intestinal obstruction site

All seven bacteria (FIG. 4B) found in retinas of Rd8 mice were formerly gastrointestinal (GI) bacteria. Therefore, metagenomic analysis was performed on microbiota in different parts of a gastrointestinal tract (including a stomach, a jejunum, an ileum, a cecum, a colon and a rectum) in WT-SPF (n=5) and Rd8-SPF (n=5) mice (FIG. 8A-F). The microbial composition in a lower digestive tract of the Rd8-SPF mice was significantly different from that in the WT-SPF mice (FIG. 8G). Akkermansia mucinphila was identified as the most distinct bacterial species in an intestinal flora of the cecum (FIG. 8H), the colon (FIG. 8I) and the rectum (FIG. 8J) of WT and Rd8 mice, although not tracked in retinas of the Rd8 mice (FIG. 4B). In fact, the seven bacteria in the retinas of the Rd8 mice were all found in a lower digestive tract of the WT and Rd8 mice, with most bacteria in the cecum (FIG. 7A). However, the bacteria were hardly detected in an upper digestive tract of the Rd8 mice. These data suggest that these retinal bacteria are present in a gastrointestinal flora of the Rd8-SPF mice rather than the WT-SPF mice.

Expression of a CRB1 protein in cecal intestinal cells of wild-type mice was identified by immunofluorescence staining, while its expression was markedly attenuated in the Rd8 mice (FIG. 9A). In addition, significant deletion of Occludin (FIG. 9B-C) expression was observed in the ceca of the Rd8 mice, while Claudin1 (FIG. 9D-E) expression was not obvious. Further transmission electron microscopy revealed normal tight and adherens junctions at a cecal epithelial barrier (FIG. 9F).

### Example 7 Examination whether barrier defects are present in colons of Rd8 mice

Similar to the results of the cecum, it was found that the CRB 1 protein was significantly expressed on the apical and basal surfaces of colonic intestinal epithelial cells (FIG. 7B). Loss of the CRB 1 protein was associated with a significant decrease in the expression of Phalloidin (FIG. 7C) and Occludin (FIG. 7D and FIG. 4E), but not with the expression of Claudin1 (FIG. 7F and FIG. 4G) protein. Importantly, by using a transmission electron microscope, it was observed that adherens junctions between colonic epithelia could not be localized in most cells, while tight junctions in Rd8 mice appeared normal (FIG. 7H and FIG. 6A). In colonic epithelia of the Rd8 mice, mitochondrial vacuolization was observed in addition to junction changes. Although the number of epithelial microvilli did not change (FIG. 7I), microvilli of the Rd8 mice became longer (FIG. 7J) and thinner (FIG. 7K), and the total number of integral adherens junctions was significantly reduced compared with WT mice (FIG. 7L). Ultrastructural changes injunctions and microvilli were also accompanied by a significant increase in the expression of Il12a in a colonic wall (FIG. 7M), while the expression of Tnfa, Il1b and mucin 2 did not change (FIG. 7N). In summary, the data suggest that the Rd8 mice have a colonic epithelial barrier defect and its associated inflammation of the colonic wall.

### Example 8 Examination whether intestinal epithelial barrier defects will lead to changes in intestinal permeability of Rd8 mice and migration of microbiota to peripheral blood flow and retinal tissue

Intestinal permeability determination was performed on WT and Rd8 mice with FICT-dextran. As shown in FIG. 11 A, there was a significant increase in fluorescence in peripheral blood of the Rd8 mice 5 hours after administration of FICT-dextran compared with the fluorescence intensity in blood of the WT mice. In addition, by using a Vancompy-labeled fecal microbiota transplantation assay, it was found that fluorescence+bacteria/cells in peripheral blood of the Rd8 mice were significantly increased compared with the WT mice 24 hours after fecal transplantation (FIG. 10B and FIG. 11B). Importantly, the appearance of Vancomopy+bacteria could be detected in retinopathy of the Rd8 mice (FIG. 11C), but not in retinopathy of the WT mice (data not shown), which was further confirmed by immunofluorescence staining of retinal tissues of the Rd8 mice (FIG. 11D). These data suggest that an intestinal tract has increased permeability to polysaccharide molecules and bacterial cells, leading to bacterial translocation from an intestinal lumen to a blood flow and retinopathy.

### Example 9 Experiment on the response of Rd8 mice to intestinal stress under the condition of increased intestinal permeability

WT and Rd8 mice were exposed to drinking water containing 1.5% dextran sulfate sodium (DSS), causing mild colitis in WT mice. After treatment with DSS for 13 days, it was found that a colon length of the Rd8 mice was significantly shorter than that of wild-type mice (FIG. 11E), and there were more bacteria in blood of the Rd8 mice than in blood of the wild-type mice, which was indicated by serum levels of bacterial 16S rRNA (FIG. 11F) and bacterial lipopolysaccharide (LPS) (FIG. 11G).

### Example 10 Reversal effect of bacteria on retinal phenotype of Rd8 mice

Although intralesional bacteria caused by disruption of an outer blood-retinal barrier and an intestinal epithelial barrier were found in retinas of Rd8 mice, it is still unclear whether these bacteria are the cause or consequence of retinal degeneration in the Rd8 mice. Therefore, the Rd8 mice were re-isolated under germ-free (GF) conditions, and it was detected whether the phenotype of retinal degeneration in the Rd8 mice was altered. As shown in FIG. 12A, retinopathy (leukoplakia) found in Rd8-SPF mice was hardly observed in germ-free Rd8 (Rd8 GF) mice. Retinal histology of the Rd8-GF mice showed that normally developing retinal tissue (FIG. 12B) did not have typical lesions found in the Rd8-SPF mice (FIG. 2A and FIG. 1C), confirming the lack of retinal degeneration in the Rd8-GF mice. The fact that retinal bacteria could not be detected in the Rd8 GF mice (FIG. 12C) was associated with a dramatic decrease in microglia in an outer nuclear layer (ONL) (FIG. 12D and FIG. 12E) of retinas in the Rd8 GF mice, and although ZO-1 and Phalloidin protein staining showed disruption of an outer limiting membrane, found in both Rd8-SPF and Rd8 GF mice (FIG. 12F). In addition, retinopathy reappeared when the Rd8 GF mice were raised in a specific pathogen-free (SPF) environment after birth (Rd8 GF SPF mice) (FIG. 12G). These data support the fact that retinal degeneration in the Rd8 mice is bacteria-dependent.

The above examples are only used to illustrate the technical solutions of the present invention, but not to limit the technical solutions of the present invention; although the present invention has been described in detail with reference to the foregoing examples, it should be understood by those of ordinary skill in the art that modifications may still be made to the technical solutions described in the foregoing examples or equivalents substitutions may still be made to some or all of the technical features in the technical solutions; and these modifications or substitutions do not make the essence of the corresponding technical solutions depart from the scope of the technical solutions of the examples of the present invention.

## Claims

1. A method for building an eye disease model or a model carrier, comprising infecting the eye disease model or the model carrier with a microorganism.

2. The method according to claim 1, **characterized in that** the infecting comprises direct contact with the microorganism or indirect contact with the microorganism, and the indirect contact with the microorganism means that a retinal barrier is present between an eye and the microorganism.

3. The method according to any one of claims 1-2, **characterized in that** the microorganism is derived from intestinal bacteria of the same individual or is the same as the intestinal bacteria of the individual.

4. The method according to any one of claims 1-3, **characterized in that** an eye disease comprises retinal degeneration; preferably, the retinal degeneration is progressive retinal degeneration; and further preferably, the retinal degeneration is inherited retinal degeneration.

5. The method according to any one of claims 1-4, **characterized in that** the eye disease comprises ocular inflammation, preferably the ocular inflammation comprises retinitis.

6. The method according to any one of claims 1-5, **characterized in that** the model is a non-human animal, preferably a monkey, a dog, a chimpanzee, a rat, and a mouse.

7. The method according to any one of claims 1-6, **characterized in that** the model carrier is selected from a cell, a tissue or an organ, the tissue or the organ is derived from a non-human animal, the cell is derived from a primary cell or a cell line of a human or a non-human animal, and the tissue or the organ is derived from a non-human animal or is derived from an ocular tissue or organ developed from human stem cells.

8. The method according to any one of claims 1-7, **characterized in that** an ocular gene in the model or the model carrier has a disease-causing mutation, preferably the ocular gene with the disease-causing mutation comprises one or two or more of the following genes with a mutation: one or a combination of two or more of ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, NMNAT1, NPHP1, NR2E3, OCA2, OTX2, PANK2, PAX6, PCARE, PCDH15, PDE6A, PDE6B, PDE6D, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RHO, RIMS1, RLBP1, ROM1, RP1, RP1L1, RP2, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC8, TULP1, USH1C, USH2A, USH2A-AS1, VAC14, VCAN, VCAN, VCAN-AS1, VHL, VPS13B, WDR19, WDR19, WDR35, WDR73, YARS1, ZFYVE26 and ZNF408.

9. The method according to any one of claims 1-8, **characterized in that** the CRB1 gene of the model or the model carrier comprises one or two or more of the following mutations: c.107C>G, c.111delT, c.135C>G, c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G>A, c.3103C>T, c.4082G> A, c.4060G>A, c.866C>T, c.1463T>C, c.2035C>G, c.2306_2307GC>AG, c.2306G>A, c.2714G>A, c.2875G>A and c.3992G>A.

10. The method according to any one of claims 1-9, **characterized in that** the CRB1 gene of the model or the model carrier comprises an Rd8 mutation.

11. The method according to any one of claims 9-10, **characterized in that** the mutation is a homozygous mutation or a heterozygous mutation.

12. The method according to any one of claims 1-11, **characterized in that** a genetic mutation existing in the model or the model carrier is innate or acquired by genetic recombination operations.

13. The method according to any one of claims 1-12, **characterized in that** the disease model has a colonic epithelial barrier defect and/or associated inflammation of a colonic wall.

14. The method according to any one of claims 1-13, **characterized in that** the microorganism is one or a combination of two or more of bacteria, archeobacteria, protists, fungi, or viruses, preferably, the microorganism is the bacteria, and the bacteria are selected from: one or two or more of *Anearostipes, Bifidobacterium, Megamonas, Nitrosomonas, Oscillibacter, Tatumella, Thiobacillus* sp., *Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, Streptomyces, Anaeroplasma,* and *Coprococcus.*

15. The method according to any one of claims 1-14, **characterized in that** the bacteria are selected from one or two or more of *Anearostipes hadrus, Bifidobacterium pseudocatenulatum, Nitrosomonas* sp.Is79A3, *Oscillibacter valericigenes, Tatumella* sp.TA1, *Megamonas funiformis, Thiobacillus denitrificans, Clostridium tetani, Clostridium perfringens, Clostridium botulinum, Acinetobacter calcoaceticus, Acinetobacter lwoffi, Acinetobacter baumannii, Acinetobacter haemolyticus, Acinetobacter junii, Acinetobacter johnsonii, Streptococcus pyogenes, Streptococcus haemolyticus, Fibrobacter succinogenes,* intestinal *Fibrobacter, Porphyromonas asacharolytica, Porphyromonas endodontalis, Porphyromonas gingivalis, Campylobacter jejuni, Campylobacter coli, Campylobacter laridis, Campylobacter upsaliensis, Campylobacter concisus, Campylobacter fetus, Actinomyces israelii, Actinomyces naeslundii, Actinomyces odontolyticus, Actinomyces viscosus, Actinomyces neuii, Escherichia coli, Escherichia blattae, Escherichia fergusonii, Escherichia hermannii, Escherichia vulneris, Tissierella praeacuta, Klebsiella pneumoniae, Klebsiella ozaenae, Azospirillum brasilense,* Achromobacter, *Thiobacillus denitrificans, Thiobacillus ferrooxidans, Thiobacillus thiooxidans,* Thiobacillus neapolitanus, *Burkholderia, Mycobacterium marinum, Treponema pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella boydii, Shigella sonnei, Frankia, Coprococcus eutactus, Streptomyces albus, Pseudomonas mendocina, Micrococcus sedentarius,* alicycline denitrifying bacteria, *Achromobacter xylosoxidans, Sphingomonas, Mycobacterium abscessus, Arthrobacter aurescens, Prevotella, Sinorhizobium meliloti,* acidic yeast, *Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Haemophilus vaginalis,* bee *Enterococcus faecium, Cytophaga hutchinsonii, Bacillus licheniformis, Xanthomonas oryzae pv. oyzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia, Lactobacillus delbrueckii, Meiothermus silvanus* (D), *Escherichia coli, Micrococcus luteus, Bacillus subtilis, Corynebacterium aurimucosum,* and *Finegoldia magna.*

16. An eye disease model carrier, **characterized in that** an eye disease is caused by infecting the model carrier with a microorganism, preferably, the model carrier is selected from a cell, a tissue or an organ, and further preferably, the cell is derived from a primary cell or a cell line of a human or a non-human animal, and the tissue or the organ is derived from a non-human animal or is derived from an ocular tissue or organ developed from human stem cells.

17. The eye disease model carrier according to claim 16, **characterized in that** the eye disease comprises retinal degeneration; preferably, the retinal degeneration is progressive retinal degeneration; and further preferably, the retinal degeneration is inherited retinal degeneration.

18. The eye disease model carrier according to any one of claims 16-17, **characterized in that** the eye disease comprises ocular inflammation, preferably the ocular inflammation comprises retinitis.

19. The eye disease model carrier according to any one of claims 16-18, **characterized in that** the microorganism is derived from intestinal bacteria of the same individual or is the same as the intestinal bacteria of the individual.

20. The eye disease model carrier according to any one of claims 16-19, **characterized in that** one or two or more of the following genes in the model carrier are mutated: one or a combination of two or more of ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

21. The eye disease model carrier according to any one of claims 16-20, **characterized in that** a mutation of the CRB1 gene of the model carrier comprises one or two or more of the following mutations: c.107C>G, c111delt, c.135C>G, c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1G>A, c.4005+2T>G, c.4006-2A>G, c.4006-1G>T, c.619G>A, c.614T>C, c.1472A>T, c.1903T>C, c.2809G>A, c.3103C>T, c.4082G> A, c.4060G>A, c.866C>T, c.1463T>C, c.2035C>G, c.2306_2307GC>AG, c.2306G>A, c.2714G>A, c.2875G>A and c.3992G>A.

22. The eye disease model carrier according to any one of claims 16-21, **characterized in that** the mutation of the CRB 1 gene of the model carrier is an Rd8 mutation.

23. The eye disease model carrier according to any one of claims 16-22, **characterized in that** the mutation is a homozygous mutation or a heterozygous mutation.

24. The eye disease model carrier according to any one of claims 16-23, **characterized in that** the mutation is is innately carried by the model carrier or acquired by gene recombination operations.

25. The eye disease model carrier according to any one of claims 16-24, **characterized in that** the non-human animal has a colonic epithelial barrier defect and/or associated inflammation of a colonic wall.

26. The eye disease model carrier according to any one of claims 16-25, **characterized in that** the microorganism is one or a combination of two or more of bacteria, archeobacteria, protists, fungi, or viruses, preferably, the microorganism is the bacteria, and the bacteria are selected from: one or two or more of *Anearostipes, Bifidobacterium, Megamonas, Nitrosomonas, Oscillibacter, Tatumella, Thiobacillus* sp., *Clostridium, Acinetobacter, Streptococcus, Mannheimia, Fibrobacter, Prevotella, Campylobacter, Actinomyces, Hymenobacter, Escherichia, Tissierella, Klebsiella, Porphyromonas, Azospira, Aquimarina, Achromobacter, Acidithiobacillus, Burkholderia, Marinobacter, Treponema, Actinosporangium, Vibrio, Ruminococcus, Methanobrevibacter, Shigella, Frankia, Streptomyces, Anaeroplasma,* and *Coprococcus.*

27. The eye disease model carrier according to any one of claims 16-26, **characterized in that** the bacteria are selected from one or two or more of *Anearostipes hadrus, Bifidobacterium pseudocatenulatum, Nitrosomonas* sp.Is79A3, *Oscillibacter valericigenes, Tatumella* sp.TA1, *Megamonas funiformis, Thiobacillus denitrificans, Clostridium tetani, Clostridium perfringens, Clostridium botulinum, Acinetobacter calcoaceticus, Acinetobacter lwoffi, Acinetobacter baumannii, Acinetobacter haemolyticus, Acinetobacter junii, Acinetobacter johnsonii, Streptococcus pyogenes, Streptococcus haemolyticus, Fibrobacter succinogenes, intestinal Fibrobacter, Porphyromonas asacharolytica, Porphyromonas endodontalis, Porphyromonas gingivalis, Campylobacter jejuni, Campylobacter coli, Campylobacter laridis, Campylobacter upsaliensis, Campylobacter concisus, Campylobacter fetus, Actinomyces israelii, Actinomyces naeslundii, Actinomyces odontolyticus, Actinomyces viscosus, Actinomyces neuii, Escherichia coli, Escherichia blattae, Escherichia fergusonii, Escherichia hermannii, Escherichia vulneris, Tissierella praeacuta, Klebsiella pneumoniae, Klebsiella ozaenae, Azospirillum brasilense, Achromobacter, Thiobacillus denitrificans, Thiobacillus ferrooxidans, Thiobacillus thiooxidans, Thiobacillus neapolitanus, Burkholderia, Mycobacterium marinum, Treponema pallidum, Treponema hyodysenteriae, Vibrio metschnikovi, Ruminococcus albus, Ruminococcus flavefaciens, Methanobrevibacter ruminantium, Shigella dysenteriae, Shigella flexneri, Shigella boydii, Shigella sonnei, Frankia, Coprococcus eutactus, Streptomyces albus, Pseudomonas mendocina, Micrococcus sedentarius, alicycline denitrifying bacteria, Achromobacter xylosoxidans, Sphingomonas, Mycobacterium abscessus, Arthrobacter aurescens, Prevotella, Sinorhizobium meliloti, acidic yeast, Staphylococcus epidermidis, Pseudomonas aeruginosa, Staphylococcus aureus, Staphylococcus haemolyticus, Pseudomonas putida, Stenotrophomonas maltophilia, Bacillus cereus, Bacillus megaterium, Lactobacillus reuteri, Haemophilus vaginalis, bee Enterococcus faecium, Cytophaga hutchinsonii, Bacillus licheniformis, Xanthomonas oryzae pv.oyzae, Acinetobacter baumannii, Acinetobacter calcoaceticus, Comamonas testosteroni, Mycobacterium kansasii, Bacillus thuringiensis, Citrobacter koseri, Dyadobacter fermentans, Serratia marcescens, Sphingomonas wittichii, Klebsiella pneumoniae, Pseudomonas fluorescens, Ralstonia pickettii, Lactobacillus crispatus, Burkholderia, Lactobacillus delbrueckii, Meiothermus silvanus (D), Escherichia coli, Micrococcus luteus, Bacillus subtilis, Corynebacterium aurimucosum,* and *Finegoldia magna.*

28. The eye disease model carrier according to any one of claims 16-27, **characterized by** being obtained according to the method of any one of claims 1-15.

29. The eye disease model carrier according to claims 16-28, **characterized by** being derived from an eye disease model built by the method according to any one of claims 1-15.

30. Application of the method according to any one of claims 1-15, an eye disease model prepared by the method according to any one of claims 1-15, or the eye disease model carrier according to any one of claims 16-29 in screening a drug for targeted treatment of an eye disease.

31. The application according to claim 30, **characterized in that** the targeted treatment targets genes related to the eye disease.

32. The application according to any one of claims 30-31, **characterized in that** the genes related to the eye disease comprise one or a combination of two or more of the following genes: ABCA4, ABCC6, ABCC9, ACBD5, ACO2, ACO2, ACTG1, ADGRV1, AHI1, AIPL1, ALMS1, AMY2B, APC, ARFGEF1, ARL13B, ARL13B, ARL6, ARMC9, ATOH7, B9D1, BAG3, BBS1, BBS1, BBS2, BBS5, BEST1, C2CD3, CA4, CABP4, CACNA1F, CBS, CC2D2A, CDH23, CDH23, CDHR1, CEMIP2, CEP104, CEP250, CEP290, CEP290, CEP41, CEP78, CERKL, CFAP410, CFAP418, CHM, CLCC1, CLCN7, CLN3, CLN5, CLN8, CLRN1, CLRN1, CNGA1, CNGA1, CNGA3, CNGB1, CNGB3, CNNM4, COL11A1, COL11A2, COL18A1, COL2A1, COL4A1, COL9A1, COL9A2, CP, CP, CPLANE1, CRB1, ERCC4, CSPP1, CTNNA1, CYP4V2, DHDDS, DYNC2H1, DYNC2I1, DYNC2I2, ENPP1, ERCC4, EVC2, EYS, EYS, F5, FAM161A, FBN1, FKRP, FKTN, FLG, FLVCR1, FOXE3, FUZ, GLB1, GMPPB, GNAT1, GRK1, GRM6, GUCA1A, GUCA1B, GUCY2D, HADHA, HGSNAT, HPS3, HPS5, IDH3B, IFT122, IFT140, IFT140, IFT43, IFT52, IFT74, IFT80, IFT80, IFT81, IFT88, IKBKG, IMPDH1, IMPG2, INPP5E, INTU, IQCB1, IQCE, IREB2, KCNJ13, KCNQ1, KCNV2, KIAA0586, KIAA0753, KIF7, KIZ, KIZ-AS1, KLHL7, KRIT1, LBR, LCA5, LOC101927157, LOC111365204, LRP2, LRP5, MAK, MAPKAPK3, MATK, MCOLN1, MERTK, MKS1, MPDZ, MT-ATP6, MT-CO3, MT-TE, MT-TL1, MTHFR, MUTYH, MYO7A, MYO7A, NMNAT1, NPHP1, NR2E3, OCA2, OTX2, PANK2, PAX6, PCARE, PCDH15, PDE6A, PDE6B, PDE6B, PDE6D, PEX1, PEX1, PEX12, PEX26, PEX6, PHF3, PITPNM3, PKD2, PLA2G5, POC5, POMT1, PRCD, PRDM13, PROM1, PRPF3, PRPF31, PRPF8, PRPH2, RAD51C, RBP3, RBP4, RD3, RDH12, RDH5, RGR, RGR, RHO, RIMS1, RLBP1, ROM1, RP1, RP1L1, RP2, RPE65, RPE65, RPGR, RPGRIP1, RPGRIP1L, RS1, SACS, SAG, SCAPER, SDCCAG8, SIX6, SLC19A1, SLC22A5, SLC26A4, SLC2A9, SLTM, SNRNP200, SPAG17, SPATA7, SPG11, TFAP2A, TGFB2, TGFBR2, TMEM107, TMEM237, TMEM67, TOGARAM1, TOPORS, TPP1, TRAF3IP1, TREX1, TRIM59-IFT80, TSPAN12, TTC21B, TTC21B, TTC8, TULP1, USH1C, USH2A, USH2A, USH2A, USH2A, USH2A-AS1, VAC14, VCAN, VCAN, VCAN-AS1, VHL, VPS13B, WDR19, WDR19, WDR35, WDR73, YARS1, ZFYVE26, ZFYVE26 and ZNF408.

33. The application according to any one of claims 30-32, **characterized in that** the targeted treatment targets one or two or more of the following mutations of the CRB1 gene: c.257_258dupTG, c.258C>T, c.428_432delGATTC, c.430T>G, c.470G>C, c.481dupG, c.482C>T, c.584G>T, c.613_619del, c.717_718insG, c.750T>G, c.915T>A, c.929G>A, c.936T>G, c.998G>A, c.1084C>T, c.1125C>G, c.1148G>A, c.1208C>G, c.1269C>A, c.1298A>G, c.1313G>A, c.1438T>C, c.1438T>G, c.1576C>T, c.1604T>C, c.1690G>T, c.1733T>A, c.1750G>T, c.1760G>A, c.1834T>C, c.1963delC, c.2025G>T, c.2042G>A, c.2128G>C, c.2129C>T, c.2185_2186insAlu, c.2219C>T, c.2222T>C, c.2234C>T, c.2245_2247del 3bp (TCA), c.2258T>C, c.2290C>T, c.2365_2367del AAT, in frame deletion, c.2401A>T, c.2438_2439ins>100A, c.2441_2442del, c.2465G>A, c.2479G>T, c.2506C>A, c.2509G>C, c.2536G>A, c.2548_2551delGGCT, c.2548G>A, c.2555T>C, c.2611_2613insT, c.2671T>G, c.2676delG, c.2681A>G, c.2688T>A, c.2816G>A, c.2843G>A, c.2853dupT, c.2884_2886delTTA, c.2957A>T, c.2966T>C, c.2983G>T, c.3002A>T, c.3008T>C, c.3035T>C, c.3037C>T, c.3074G>A, c.3074G>T, c.3122T>C, c.3212T>C, c.3296C>A, c.3299T>C, c.3299T>G, c.3307G>A/C, c.3320T>C, c.3320T>G, c.3331G>T, c.3343_3352del, c.3347delT, c.3343_3352del, c.3347delT, c.3427delT, c.3482A>G, c.3493T>C, c.3655T>G, c.3541T>C, c.3542dupG, c.3593A>G, c.3613G>A, c.3653G>T, c.3659_3660delinsA, c.3664C>T, c.3668G>C, c.3676G>T, c.3713_3716dup, c.3879G>A, c.3914C>T, c.3949A>C, c.3961T>A, c.3988delG, c.3988G>T, c.3995G>T, c.3996C>A, c.3997G>T, c.4094C>A, c.4121_4130del, c.4142C>T, c.4148G>A, c.2128+2T>G, c.2842+5G>A, c.3878+1G>T, c.4005+1 G>A, c.4005+2T>G, c.4006-2A>G, c.4006-1G>T, c.619G>A, c.614T>C, c.1472A>T, c.1903T>C, c.2809G>A, c.3103C>T, c.4082G> A, c.4060G>A, c.866C>T, c.1463T>C, c.2035C>G, c.2306_2307GC>AG, c.2306G>A, c.2714G>A, c.2875G>A and c.3992G>A.

34. The application according to any one of claims 30-32, **characterized in that** the drug for targeted treatment comprises modified cells, modified proteins, RNA targeting the genes according to claim 32 or targeting sites of the mutations according to claim 33 and/or DNA targeting the genes according to claim 32 or targeting sites of the mutations according to claim 33.

35. Application of the method according to any one of claims 1-15, an eye disease model prepared by the method according to any one of claims 1-15, or the eye disease model carrier according to any one of claims 16-29 in research related to an eye disease.

36. Application of the method according to any one of claims 1-15, an eye disease model prepared by the method according to any one of claims 1-15, or the eye disease model carrier according to any one of claims 16-29 in screening a drug related to an eye disease, the drug comprising one or a combination of two or more of a small molecule drug, a chemical drug, a high molecular drug, a biological drug or a natural drug (e.g., a traditional Chinese medicine or a traditional Chinese medicine extract), a cellular drug, an RNA drug, and a DNA drug.
